# EUROPÄISCHE PATENTSCHRIFT

(11) **EP 1 098 864 B1**
(45) Veröffentlichungstag und Bekanntmachung des Hinweises auf die Patenterteilung: **06.10.2004**
(21) Anmeldenummer: 99934714.9
(22) Anmeldetag: 20.07.1999
(51) Int. Cl.: C07B 35/02, C07D 295/02

(54) **VERFAHREN ZUR HYDRIERUNG VON UNGESÄTTIGTEN HETEROCYCLISCHEN VERBINDUNGEN**
METHOD FOR HYDROGENATING UNSATURATED HETEROCYCLIC COMPOUNDS
PROCEDE D'HYDROGENATION DE COMPOSES HETEROCYCLIQUES NON SATURES

(30) Priorität: 21.07.1998 DE 19832810
(43) Veröffentlichungstag der Anmeldung: 16.05.2001
(73) Patentinhaber: BASF AKTIENGESELLSCHAFT, 67056 Ludwigshafen (DE)
(72) Erfinder: BÖTTCHER, Arnd, D-67227 Frankenthal (DE); BRUNNER, Melanie, D-67105 Schifferstadt (DE); HENKELMANN, Jochem, D-68165 Mannheim (DE); RÜTTER, Heinz, D-67126 Hochdorf-Assenheim (DE); BREITSCHEIDEL, Boris, D-67117 Limburgerhof (DE)
(74) Vertreter: Isenbruck, Günter, Dr.
(86) Internationale Anmeldenummer: PCT/EP1999/005178
(87) Internationale Veröffentlichungsnummer: WO 2000/005184

(56) Entgegenhaltungen:
- EP-A- 0 813 906
- EP-A- 0 814 098
- DE-A- 1 620 664
- DE-A- 2 550 716
- US-A- 2 843 589
- US-A- 4 083 851

## Beschreibung

Die vorliegende Erfindung betrifft ein Verfahren zur Hydrierung von ungesättigten heterocyclischen Verbindungen durch Inkontaktbringen einer oder mehrerer ungesättigter heterocyclischer Verbindungen, insbesondere von Pyridin oder Pyridinderivaten, mit einem Wasserstoff enthaltenden Gas in Gegenwart eines Makroporen aufweisenden Katalysators.

Die DE-A 16 20 664 betrifft ein Verfahren zur Herstellung von Piperidin und seinen Alkylhomologen durch katalytische Hydrierung von Pyridin und dessen Alkylhomologen in Gegenwart von Nickel-Katalysatoren, wobei die Hydrierung bei Temperaturen von 100 bis 200 °C und Drücken bis zu 200 bar durchgeführt wird, wobei die verwendeten Katalysatoren mindestens 30 Gew.-% Nickel auf aktivem Al₂O₃ oder Chromoxid enthalten.

In der JP 52 148 083 wird die Herstellung von Piperidin aus Pyridin bei 100 bis 200 °C in Gegenwart von Ruthenium-haltigen Hydrierkatalysatoren beschrieben. Die dort beschriebenen Katalysatoren werden vor der Hydrierung mit schwefelhaltigen Verbindungen, wie z.B. Mercaptanen, Thiophenen und Sulfolanen vorbehandelt. Die dort beschriebene Vorbehandlung mit schwefelhaltigen Verbindungen ist jedoch vergleichsweise aufwendig und wirkt sich negativ auf die Hydrieraktivität der dort verwendeten Katalysatoren aus.

Pyridin läßt sich auch über einem geträgerten Ruthenium-Katalysator, der als Träger Kohle oder Y-Al₂O₃ und einen Gehalt an Ruthenium von 5% aufweist, bei aufweist, bei 80 bis 130 °C und 20 bis 120 bar zu Piperidin hydrieren, wie dies in der SU 255 940 beschrieben wird. Gemäß des dort beschriebenen Verfahrens sind jedoch zur Erzielung hoher Hydrieraktivitäten hohe Metallbeladungen von 5 Gew.-% oder mehr notwendig.

In ähnlicher Weise können 2-Alkylpiperidine auch ohne Verwendung eines Lösungsmittels hergestellt werden, indem ein Katalysator eingesetzt wird, der 0,1 bis 10 Gew.-% Ruthenium oder RuO₂ auf Al₂O₃ oder Aktivkohle enthält, wie dies in der DE-A 25 50 716 beschrieben wird. Die Hydrierung gemäß dieser Druckschrift wird bei Drücken < 50 bar und einer Temperatur von 160 bis 180 °C durchgeführt. Dieses Verfahren zeigt jedoch eine lediglich ungenügende Selektivität. Ein weiterer Nachteil liegt darin, daß die Verwendung dieses Verfahren auf alkylsubstituierte Pyridine beschränkt ist, unter den dort angegebenen Bedingungen unter Verwendung des dort beschriebenen Katalysators kann Pyridin nicht zü Piperidin hydriert werden.

US Patent US 4,083,851 offenbart ein Verfahren zur Herstellung von cis-2,5-Dialkylpyrrolidinen aus 2,5-Dialkylpyrrolen oder 2,5-Dialkylpyrrolinen durch katalytische Hydrierung mit einem Ruthenium-Katalysator auf einem Aluminiumoder Titanoxid-Träger.

Die US 2,843,589 betrifft eine Methode zur katalytischen Hydrierung von Pyrazinen zur Herstellung von Piperazin und Piperazinderivaten. Die Reaktion wird katalysiert durch Raney Nickel, geträgertes reduziertes Nickel, geträgertes Rhodium oder Palladium bei einer Temperatur von 125 bis 225 °C und einem Druck von 200 bis 600 psi.

Demgemäß lag eine Aufgabe der vorliegenden Erfindung in der Bereitstellung eines verbesserten Verfahrens zur Selektivhydrierung von ungesättigten heterocyclischen Verbindungen, insbesondere von Pyridin bzw. dessen Homologen, zu Piperidin bzw. dessen Homologen, wobei sehr hohe Ausbeuten bzw. nahezu vollständiger Umsatz erreicht werden sollte.

Eine weitere Aufgabe der vorliegenden Erfindung lag in der Bereitstellung eines derartigen Verfahrens, wobei lediglich ein minimaler Anteil von Nebenprodukten bzw. Zersetzungsprodukten während der Hydrierung anfallen sollte. Ferner sollte es mit dem erfindungsgemäßen Verfahren möglich sein, dieses unter hohen Katalysatorbelastungen und langen Standzeiten mit einer extrem hohen Turn-Over-Zahl durchzuführen, wobei die entsprechenden Hydrierungsprodukte in hoher Ausbeute und hoher Reinheit erhalten werden sollten.

Diese und weitere Aufgaben werden durch das gemäß dieser Anmeldung bereitgestellte Verfahren gelöst.

Demgemäß betrifft die vorliegende Erfindung ein Verfahren zur Hydrierung von Pyridin oder Alkylpyridinen oder eines Gemischs aus zwei oder mehr davon durch Inkontaktbringen von Pyridin oder Alkylpyridinen oder des Gemischs aus zwei oder mehr davon mit einem Wasserstoff enthaltenden Gas in Gegenwart eines Katalysators, der als Aktivmetall, aufgebracht auf einem Träger, Ruthenium alleine oder gemeinsam mit mindestens einem Metall der Übergangsgruppe I oder VII des Periodensystems, wobei 10 bis 50 % des Porenvolumens des Trägers von Makroporen mit einem Porendurchmesser im Bereich von 50 nm bis 10.000 nm und 50 bis 90 % des Porenvolumens des Trägers von Mesoporen mit einem Porendurchmesser im Bereich von 2 bis 50 nm gebildet werden, wobei sich die Summe der Porenvolumina zu 100% addiert.

In einer bevorzugten Ausführungsform betrifft die vorliegende Erfindung ein Verfahren wie oben definiert, das dadurch gekennzeichnet ist, daß der Katalysator als Aktivmetall Ruthenium alleine oder zusammen mit mindestens einem Metall der I. oder VII. Nebengruppe des Periodensystems, aufgebracht auf einem Träger, umfaßt, wobei der Träger einen mittleren Porendurchmesser von mindestens 50 nm und eine Oberfläche BET von höchstens 30 m²/g aufweist und die Menge des Aktivmetalls 0,01 bis 30 Gew.- %, bezogen auf das Gesamtgewicht des Katalysator, beträgt.

Ferner betrifft sie ein derartiges Verfahren, wobei der Katalysator als Aktivmetall Ruthenium alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt.

In einer weiteren bevorzugten Ausführungsform betrifft die vorliegende Erfindung ein Verfahren wie oben definiert, wobei der Katalysator als Aktivmetall Ruthenium alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einen Träger, umfaßt, wobei der Träger einen mittleren Porendurchmesser von mindestens 0,1 µm, und eine Oberfläche BET von höchstens 15 m²/g aufweist.

Als Aktivmetall wird Ruthenium verwendet. Unter den ebenfalls verwendbaren Metallen der I. oder VII. Nebengruppe des Periodensystems, die ebenfalls allesamt prinzipiell verwendbar sind, werden vorzugsweise Kupfer und/oder Rhenium eingesetzt.

Die Begriffe "Makroporen" und "Mesoporen" werden im Rahmen der vorliegenden Erfindung so verwendet, wie sie in Pure Appl. Chem., 45, S. 79 (1976) definiert sind, nämlich als Poren, deren Durchmesser oberhalb von 50 nm (Makroporen) oder deren Durchmesser zwischen 2 nm und 50 nm liegt (Mesoporen).

Der Gehalt des Aktivmetalls beträgt im allgemeinen 0,01 bis 30 Gew.-%, vorzugsweise 0,01 bis 5 Gew.-% und insbesondere 0,1 bis 5 Gew.-%, jeweils bezogen auf das Gesamtgewicht des verwendeten Katalysators, wobei die bei den im folgenden beschriebenen, vorzugsweise eingesetzten Katalysatoren 1 bis 3 vorzugsweise verwendeten Gehalte nochmals bei der Diskussion dieser Katalysatoren einzeln angegeben sind.

Es lassen sich auch mit nicht reduzierbaren Gruppen substituierte Verbindungen der oben genannten Art, wie Alkylpyridine, hydrieren.

Die jeweils eingesetzten Verbindungen werden dann selektiv zu den entsprechenden kernhydrierten Verbindungen umgesetzt.

Mit dem vorliegenden Verfahren wird die folgende Umsetzung durchgeführt:
- Umsetzung von Pyridin zu Piperidin.

Die dabei im allgemeinen verwendeten Reaktionsparameter werden untenstehend im Abschnitt "Die Verfahrensführung" nochmals kurz erläutert.

Im folgenden soll nunmehr der vorzugsweise verwendete Katalysator detailliert beschrieben werden. Dabei erfolgt die Beschreibung unter Bezugnahme auf die Verwendung von Ruthenium als Aktivmetall.

### KATALYSATOR,

Die erfindungsgemäß verwendeten Katalysatoren enthalten Ruthenium als Aktivkomponente auf einem Träger, wie hierin definiert.

Der erfindungsgemäß verwendete Katalysator kann technisch hergestellt werden durch Auftragen von Ruthenium und gegebenenfalls mindestens einem Metall der I. oder VII. Nebengruppe des Periodensystems auf
einem geeigneten Träger. Die Auftragung kann durch Tränken des Trägers in wäßrigen Metallsalzlösungen, wie z.B. Rutheniumsalzlösungen, durch Aufsprühen entsprechender Metallsalzlösungen auf den Träger oder durch andere geeignete Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorkomplexe, Nitritokomplexe oder Aminkomplexe der entsprechenden Metalle, wobei die Nitrate und Nitrosylnitrate bevorzugt sind.

Bei Katalysatoren, die mehrere Aktivmetalle auf den Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend getrocknet, wobei Temperaturen zwischen 100°C und 150°C bevorzugt sind. Wahlweise können diese Träger bei Temperaturen zwischen 200°C und 600°C, vorzugsweise 350°C bis 450°C calciniert werden. Anschließend werden die beschichteten Träger durch Behandlung in einem Gasstrom, der freien Wasserstoff enthält, bei Temperaturen zwischen 30°C und 600°C, vorzugsweise zwischen 100°C und 450°C und insbesondere 100°C bis 300°C aktiviert. Der Gasstrom besteht vorzugsweise aus 50 bis 100 Vol.-% H₂ und 0 bis 50 Vol.-% N₂.

Werden auf die Träger mehrere Aktivmetalle aufgetragen und erfolgt das Auftragen nacheinander, so kann der Träger nach jedem Auftragen bzw. Tränken bei Temperaturen zwischen 100°C und 150°C getrocknet werden und wahlweise bei Temperaturen zwischen 200°C und 600°C calciniert werden. Dabei kann die Reihenfolge, in der die Metallsalzlösung aufgetragen oder aufgetränkt werden, beliebig gewählt werden.

Die Metallsalzlösung wird in einer solchen Menge auf den/die Träger aufgebracht, daß der Gehalt an Ruthenium 0,01 bis 30 Gew.-%, vorzugsweise 0,01 bis 10 Gew.-%, weiter bevorzugt 0,01 bis 5 Gew.-%, und insbesondere 0,3 bis 1 Gew. - % bezogen auf das Gesamtgewicht des Katalysators, beträgt.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemisorptionsverfahien gemessen, wie es in J. LeMaitre et al., *"Characterization of Heterologous Catalysts",* Hrsg. Francis Delanney, Marcel Dekker, New York (1984), S. 310 - 324, beschrieben ist.

Im erfindungsgemäß verwendeten Katalysator 2 beträgt das Verhältnis der Oberflächen des mindestens einen Aktivmetalls und des Katalysatorträgers weniger als ungefähr 0,3, vorzugsweise weniger als ungefähr 0,1 und insbesondere ungefähr 0,05 oder weniger, wobei der untere Grenzwert bei ungefähr 0,0005 liegt.

Die zur Herstellung der erfindungsgemäß verwendeten Katalysatoren 2 verwendbaren Trägermaterialien besitzen Makroporen und Mesoporen.

Dabei weisen die erfindungsgemäß verwendbaren Träger eine Porenverteilung auf, der gemäß 10 bis 50%, vorzugsweise 10 bis 45%, weiter bevorzugt 10 bis 30 und insbesondere 15 bis 25 % des Porenvolumens von Makroporen mit Porendurchmesser im Bereich von 50 nm bis 10.000 nm und 50 bis 90%, vorzugsweise 55 bis 90%, weiter bevorzugt 70 bis 90% und insbesondere 75 bis 95% des Porenvolumens von Mesoporen mit einem Porendurchmesser von 2 bis 50 nm gebildet werden, wobei sich jeweils die Summe der Porenvolumina zu 100% addiert.

In einer weiteren Ausführungsform werden Trägermaterialien verwendet, die makroporös sind und einen mittleren Porendurchmesser von mindestens 50 nm, vorzugsweise mindestens 100 nm, insbesondere mindestens 500 nm, aufweisen und deren Oberfläche nach BET höchstens 30 m²/g, vorzugsweise höchstens 15 m²/g, weiter bevorzugt höchstens 10 m²/g, insbesondere höchstens 5 m²/g und weiter bevorzugt höchstens 3 m²/g liegt. Der mittlere Porendurchmesser des Trägers beträgt vorzugsweise 100 nm bis 200 µm, weiter bevorzugt 500 nm bis 50 µm. Die Oberfläche des Trägers beträgt vorzugsweise 0,2 bis ungefähr 15 m²/g, weiter bevorzugt 0,5 bis 10 m²/g, insbesondere 0,5 bis 5 m²/g und weiter bevorzugt 0,5 bis 3 m²/g.

Weiterhin sind die verwendbaren Trägermaterialien vorzugsweise solche, die makroporös sind und einen mittleren Porendurchmesser von mindestens 0,1 µm, vorzugsweise mindestens 0,5 µm, und eine Oberfläche von höchstens 15 m²/g aufweisen, vorzugsweise höchstens 10 m²/g, besonders bevorzugt höchstens 5 m²/g, insbesondere höchstens 3 m²/g.

Das Gesamtporenvolumen der erfindungsgemäß verwendeten Träger beträgt 0,05 bis 1,5 cm³/g, vorzugsweise 0,1 bis 1,2 cm³/g und insbesondere 0,3 bis 1,0 cm³/g. Der mittlere Porendurchmesser der erfindungsgemäß verwendeten Träger beträgt 5 bis 20 nm, vorzugsweise 8 bis 15 nm und insbesondere 9 bis 12 nm.

Vorzugsweise beträgt die Oberfläche des Trägers 50 bis 500 m²/g, weiter bevorzugt 200 bis 350 m²/g und insbesondere 250 bis 300 m²/g des Trägers.

Die Oberfläche des Trägers wird nach dem BET-Verfahren durch N₂-Adsorption, insbesondere nach DIN 66131, bestimmt. Die Bestimmung des mittleren Porendurchmesser und der Größenverteilung erfolgt durch Hg-Porosimetrie, insbesondere nach DIN 66133.

Obwohl prinzipiell alle bei der Katalysatorherstellung bekannten Trägermaterialien, d.h. die die oben definierte Porengrößenverteilung aufweisen, eingesetzt werden können, werden vorzugsweise Aktivkohle, Siliciumcarbid, Aluminiumoxid, Siliciumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

Weitere Details bezüglich des Katalysators bzw. zu seiner Herstellung sind der DE-A 196 24 485.4 zu entnehmen, deren diesbezüglicher Inhalt durch Bezugnahme vollständig in die vorliegende Anmeldung einbezogen wird.

### DIE VERFAHRENSFÜHRUNG

Die erfindungsgemäß durchgeführte Hydrierung wird bei geeigneten Drücken und Temperaturen durchgeführt. Bevorzugt sind dabei Drücke oberhalb von 10 bar, vorzugsweise von 20 bis 300 bar. Die während der Hydrierung eingesetzten Temperaturen betragen 50 bis 120 °C, bevorzugt 80 bis 120 °C.

Das erfindungsgemäße Verfahren kann entweder kontinuierlich oder diskontinuierlich durchgeführt werden, wobei die kontinuierliche Verfahrensdurchführung bevorzugt ist.

Bei der kontinuierlichen Verfahrensführung beträgt die Menge der zur Hydrierung vorgesehenen heterocyclischen Verbindung bzw. des Gemischs aus zwei oder mehr

Die erfindungsgemäße Hydrierung kann in Ab- oder Anwesenheit eines Lösungsoder Verdünnungsmittels durchgeführt werden, d.h. es ist nicht erforderlich, die Hydrierung in Lösung durchzuführen.

Als Lösungs- oder Verdünnungsmittel kann jedes geeignete Lösungsmittel- oder Verdünnungsmittel eingesetzt werden. Die Auswahl ist dabei nicht kritisch, solange das eingesetzte Lösungs- oder Verdünnungsmittel in der Lage ist, mit der zu hydrierenden heterocyclischen Verbindung eine homogene Lösung zu bilden. Beispielsweise können die Lösungs- oder Verdünnungsmittel auch Wasser enthalten.

### Beispiele geeigneter Lösungs- oder Verdünnungsmittel schließen die folgenden ein:

Geradkettige oder cyclische Ether, wie beispielsweise Tetrahydrofuran oder Dioxan, sowie aliphatische Alkohole, in denen der Alkylrest vorzugsweise 1 bis 10 Kohlenstoffatome, insbesondere 3 bis 6 Kohlenstoffatome, aufweist.

Beispiele bevorzugt verwendbarer Alkohole sind i-Propanol, n-Butanol, i-Butanol und n-Hexanol.

Im folgenden soll nunmehr das erfindungsgemäße Verfahren anhand einiger Ausführungsbeispiele näher erläutert werden.

### BEISPIELE

### Herstellung Katalysator A

Ein meso-/makroporöser Aluminiumoxidträger in Form von 3 bis 5 mm-Kugeln mit einem Gesamtporenvolumen von 0,44 cm³/g, wobei 0,09 cm³/g (20% des Gesamtporenvolumens) von Poren mit einem Durchmesser im Bereich von 50 nm

### BEISPIELE

### Herstellung Katalysator A

Ein meso-/makroporöser Aluminiumoxidträger in Form von 3 bis 5 mm-Kugeln mit einem Gesamtporenvolumen von 0,44 cm³/g, wobei 0,09 cm³/g (20% des Gesamtporenvolumens) von Poren mit einem Durchmesser im Bereich von 50 nm bis 10.000 nm und 0,35 cm³/g (80% des Gesamtporenvolumens) von Poren mit einem Durchmesser im Bereich von 2 nm bis 50 nm gebildet werden, einem mittleren Porendurchmesser von 11 nm und einer Oberfläche von 268 m²/g wurde mit einer wäßrigen Ruthenium-(III)-nitrat-Lösung getränkt. Das während des Tränkens vom Träger aufgenommene Lösungsvolumen entsprach dabei in etwa dem Porenvolumen des verwendeten Trägers. Anschließend wurde der mit der Ruthenium-(III)-nitrat-Lösung getränkte Träger bei 120 °C getrocknet und bei 200 °C im Wasserstrom aktiviert (reduziert). Der so hergestellte Katalysator enthielt 0,5 Gew.-% Ruthenium, bezogen auf das Gewicht des Katalysators. Die Rutheniumoberfläche betrug 0,72 m²/g, das Verhältnis von Ruthenium- zu Trägeroberfläche lag bei 0,0027.

### Vergleichsbeispiel 1

In einem 300 ml-Druckreaktor wurden 8 g Katalysator A in einem Korbeinsatz vorgelegt. Anschließend wurde der Reaktor mit 100 g Pyridin befüllt. Die Hydrierung wurde mit reinem Wasserstoff bei einem Druck von 150 bis 250 bar und einer Temperatur von 180 °C durchgeführt. Es wurde so lange hydriert, bis kein Wasserstoff mehr aufgenommen wurde (0,5 h) und der Reaktor anschließend entspannt. Der Umsatz des Pyridins betrug 100%. Die Ausbeute an Piperidin lag bei 99 %

### Beispiel 1

In einem 300 ml-Druckreaktor wurden 8 g Katalysator A in einem Korbeinsatz vorgelegt. Anschließend wurde der Reaktor mit 40 g Pyridin und 80 ml THF befüllt. Die Hydrierung wurde mit reinem Wasserstoff bei einem Druck von 200 bar und einer Temperatur von 120 °C durchgeführt. Es wurde so lange hydriert, bis kein Wasserstoff mehr aufgenommen wurde (2h) und der Reaktor anschließend entspannt. Der Umsatz des Pyridins betrug 100%. Die Ausbeute an Piperidin lag bei 98,6%.

### Beispiel 2

In einem 300 ml-Druckreaktor wurden 8 g Katalysator A in einem Korbeinsatz vorgelegt. Anschließend wurde der Reaktor mit 50 g Pyridin und 80 ml THF befüllt. Die Hydrierung wurde mit reinem Wasserstoff bei einem Druck von 50 bar und einer Temperatur von 120 °C durchgeführt. Es wurde so lange hydriert, bis kein Wasserstoff mehr aufgenommen wurde (3h) und der Reaktor anschließend entspannt. Der Umsatz des Pyridins betrug 100%. Die Ausbeute an Piperidin lag bei 98,3%.

### Beispiel 3

In einem 300 ml-Druckreaktor wurden 8 g Katalysator A in einem Korbeinsatz vorgelegt. Anschließend wurde der Reaktor mit 40 g Pyridin und 80 ml THF befüllt. Die Hydrierung wurde mit reinem Wasserstoff bei einem Druck von 10 bar und einer Temperatur von 120 °C durchgeführt. Es wurde so lange hydriert, bis kein Wasserstoff mehr aufgenommen wurde (12h) und der Reaktor anschließend entspannt. Der Umsatz des Pyridins betrug 100%. Die Ausbeute an Piperidin lag bei 98.1%.

### Vergleichsbeispiel 2

1,2 l Katalysator A wurden in einen elektrisch beheizbaren Durchflußreaktor eingefüllt. Anschließend wurde ohne vorhergehende Aktivierung bei 2 x 10⁷ Pa und 160 °C mit der Hydrierung von Pyridin begonnen. Die Hydrierung wurde kontinuierlich in Sumpffahrweise ausgeführt, wobei ein Teil des Hydrierungsaustrags über eine Umlaufpumpe rückgeführt und dem Einsatzstoff vor dem Reaktor zugemischt wurde. Bezogen auf die Menge an Pyridin wurde so die zehnfache Menge an Hydrierungsprodukt als Lösungsmittel zugesetzt. Am Kopf des Abscheiders wurden 500 bis 600 l H₂/h entspannt. Die kontinuierlich dem Reaktor zugeführte Pyridinmenge entsprach einer Katalysatorbelastung von 0,3 kg/l x h.

In Abhängigkeit von den Reaktionstemperaturen ergaben sich bei stationären Reaktionsbedingungen folgende durch Gaschromatographie bestimmte Produktzusammensetzungen:

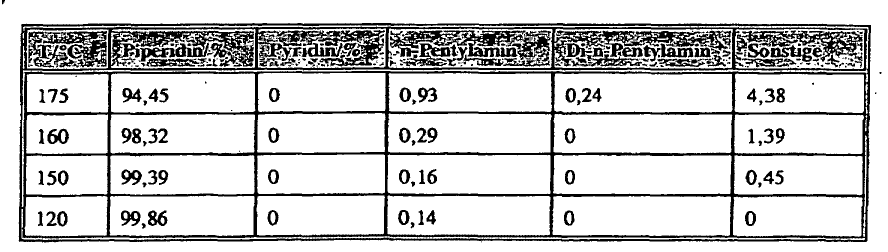

Die Katalysatorstandzeit unter den gewählten Bedingungen betrug mindestens 1000 h, ohne daß eine Aktivitäts- und Selektivitätsabnahme beobachtet werden konnte.

## Patentansprüche

1. Verfahren zur Hydrierung von Pyridin, eines Alkylpyridins oder eines Gemischs aus zwei oder mehr davon durch Inkontaktbringen von Pyridin oder des Alkylpyridins oder des Gemischs aus zwei oder mehr davon mit einem Wasserstoff enthaltenden Gas bei einer Temperatur im Bereich von 50 °C bis 120 °C in Gegenwart eines Katalysators, der als Aktivmetall, aufgebracht auf einem Träger, Ruthenium alleine oder gemeinsam mit mindestens einem Metall der Übergangsgruppe I oder VII des Periodensystems umfasst, wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Porenvolumina zu 100 % addiert.

2. Verfahren nach Anspruch 1, **dadurch gekennzeichnet, dass** der Katalysator als Aktivmetall Ruthenium alleine oder zusammen mit mindestens einem Metall der I. oder VII. Nebengruppe des Periodensystems, aufgebracht auf einem Träger, umfasst, wobei der Träger einen mittleren Porendurchmesser von mindestens 50 nm und eine Oberfläche BET von höchstens 30 m²/g aufweist und die Menge des Aktivmetalls 0,01 bis 30 Gew.- %, bezogen auf das Gesamtgewicht des Katalysator, beträgt.

3. Verfahren nach Anspruch 1, **dadurch gekennzeichnet, dass** der Katalysator als Aktivmetall Ruthenium alleine oder zusammen mit mindestens einem Metall der L oder VII. Nebengruppe des Periodensystems in einer Menge von 0,01 bis 30 Gew.-%, bezogen auf das Gesamtgewicht des Katalysators, aufgebracht auf einem Träger, umfasst

4. Verfahren nach Anspruch 1, **dadurch gekennzeichnet, dass** der Katalysator als Aktivmetall Ruthenium alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einen Träger, umfasst, wobei der Träger einen mittleren Porendurchmesser von mindestens 0,1 µm, und eine Oberfläche BET von höchstens 15 m²/g aufweist.

5. Verfahren nach einem der vorstehenden Ansprüche, **dadurch gekennzeichnet, dass** der Träger Aktivkohle, Siliciumcarbid, Aluminiumoxid, Siliciumdioxid, Titandioxid, Zirkoniumdioxid, Magnesiumoxid, Zinkoxid oder ein Gemisch aus zwei oder mehr davon enthält.

6. Verfahren nach einem der vorstehenden Ansprüche, **dadurch gekennzeichnet, dass** die Hydrierung in Gegenwart eines Lösungs- oder Verdünnungsmittels durchgeführt wird.

7. Verfahren nach einem der vorstehenden Ansprüche, **dadurch gekennzeichnet, dass** die Hydrierung kontinuierlich durchgeführt wird.

## Claims

1. A process for hydrogenating pyridine, an alkylpyridine or a mixture of two or more thereof by contacting pyridine or the alkylpyridine or the mixture of two or more thereof with a hydrogencontaining gas at from 50°C to 120°C in the presence of a catalyst comprising as active metal, applied to a support, ruthenium alone or together with at least one metal from transition group I or VII of the Periodic Table, from 10 to 50 % of the pore volume of the support being formed by macropores having a pore diameter in the range from 50 nm to 10,000 nm and from 50 to 90 % of the pore volume of the support being formed by mesopores having a pore diameter in the range from 2 to 50 nm, the sum of the pore volumes adding to 100 %.

2. A process as claimed in claim 1, wherein the catalyst comprises as active metal ruthenium alone or together with at least one metal from transition group I or VII of the Periodic Table, applied to a support, the support having an average pore diameter of at least 50 nm and a BET surface area of not more than 30 m²/g and the amount of active metal being from 0.01 to 30 % by weight, based on the overall weight of the catalyst.

3. A process as claimed in claim 1, wherein the catalyst comprises as active metal ruthenium alone or together with at least one metal from transition group I or VII of the Periodic Table in an amount of from 0.01 to 30 % by weight, based on the overall weight of the catalyst, applied to a support.

4. A process as claimed in claim 1, wherein the catalyst comprises as active metal ruthenium alone or together with at least one metal from transition group I or VII of the Periodic Table in an amount of from 0.01 to 30 % by weight, based on the overall weight of the catalyst, applied to a support, the support having an average pore diameter of at least 0.1 µm and a BET surface area of not more than 15 m²/g.

5. A process as claimed in any of the preceding claims, wherein the support comprises activated carbon, silicon carbide, alumina, silica, titanium dioxide, zirconium oxide, magnesium oxide, zinc oxide or a mixture of two or more thereof.

6. A process as claimed in any of the preceding claims, wherein the hydrogenation is conducted in the presence of a solvent or diluent.

7. A process as claimed in any of the preceding claims, wherein the hydrogenation is conducted continuously.

## Revendications

1. Procédé d'hydrogénation de pyridine, d'une alkylpyridine ou d'un mélange d'au moins deux d'entre elles, par mise en contact de pyridine ou de l'alkylpyridine ou du mélange d'au moins deux d'entre elles avec un gaz contenant de l'hydrogène, à une température comprise dans la plage de 50 à 120°C en présence d'un catalyseur qui comprend en tant que métal actif appliqué sur un support du ruthénium seul ou en même temps qu'au moins un métal du Ier ou du VIIème groupe secondaire du Tableau Périodique, 10 à 50 % du volume des pores du support étant constitués de macropores ayant un diamètre de pore compris dans la plage de 50 à 10 000 nm, et 50 à 90 % du volume des pores du support étant constitués de mésopores ayant un diamètre de pore compris dans la plage de 2 à 50 nm, la somme des volumes des pores faisant 100 %.

2. Procédé selon la revendication 1, **caractérisé en ce que** le catalyseur comprend en tant que métal actif du ruthénium seul ou en même temps qu'au moins un métal du Ier ou du VIIème groupe secondaire du Tableau Périodique, appliqué sur un support, le support ayant un diamètre moyen des pores d'au moins 50 nm et une aire BET d'au plus 30 m²/g, la quantité du métal actif étant de 0,01 à 30 % en poids par rapport au poids total du catalyseur.

3. Procédé selon la revendication 1, **caractérisé en ce que** le catalyseur comprend en tant que métal actif du ruthénium seul ou en même temps qu'au moins un métal du Ier ou du VIIème groupe secondaire du Tableau Périodique en une quantité de 0,01 à 30 % en poids par rapport au poids total du catalyseur, appliqué sur un support.

4. Procédé selon la revendication 1, **caractérisé en ce que** le catalyseur comprend en tant que métal actif du ruthénium seul ou en même temps qu'au moins un métal du Ier ou du VIIème groupe secondaire du Tableau Périodique en une quantité de 0,01 à 30 % en poids par rapport au poids total du catalyseur, appliqué sur un support, le support ayant un diamètre moyen des pores d'au moins 0,1 µm et une aire BET d'au plus 15 m²/g.

5. Procédé selon l'une des revendications précédentes, **caractérisé en ce que** le support contient du charbon actif, du carbure de silicium, de l'oxyde d'aluminium, du dioxyde de silicium, du dioxyde de titane, du dioxyde de zirconium, de l'oxyde de magnésium, de l'oxyde de zinc ou un mélange d'au moins deux d'entre eux.

6. Procédé selon l'une des revendications précédentes, **caractérisé en ce que** l'hydrogénation est mise en oeuvre en présence d'un solvant ou d'un diluant.

7. Procédé selon l'une des revendications précédentes, **caractérisé en ce que** l'hydrogénation est mise en oeuvre en continu.
